(19) Europäisches Patentamt / European Patent Office / Office européen des brevets

(11) **EP 2 231 528 B1**

(12) **EUROPÄISCHE PATENTSCHRIFT**

(45) Veröffentlichungstag und Bekanntmachung des Hinweises auf die Patenterteilung:
**03.07.2013 Patentblatt 2013/27**

(21) Anmeldenummer: **08862319.4**

(22) Anmeldetag: **19.12.2008**

(51) Int Cl.:
*C02F 1/68* (2006.01)     *C02F 3/28* (2006.01)
*C02F 11/04* (2006.01)

(86) Internationale Anmeldenummer:
**PCT/EP2008/068115**

(87) Internationale Veröffentlichungsnummer:
**WO 2009/077623 (25.06.2009 Gazette 2009/26)**

(54) **SPURENELEMENTLÖSUNG FÜR BIOGASVERFAHREN**

TRACE ELEMENT SOLUTION FOR BIOGAS METHODS

SOLUTION D'OLIGOÉLÉMENT POUR PROCÉDÉ AU BIOGAZ

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **19.12.2007 DE 102007061138**

(43) Veröffentlichungstag der Anmeldung:
**29.09.2010 Patentblatt 2010/39**

(60) Teilanmeldung:
**13154439.7 / 2 592 055**

(73) Patentinhaber: **Agraferm Technologies AG**
**85276 Pfaffenhofen/Ilm (DE)**

(72) Erfinder:
• **FRIEDMANN, Hans**
  **85298 Scheyern-Fernhag (DE)**
• **KUBE, Jürgen**
  **85276 Pfaffenhofen (DE)**

(74) Vertreter: **Ganahl, Bernhard et al**
**PATRONUS IP**
**Patent- und Rechtsanwälte**
**Truderinger Straße 246**
**81825 München (DE)**

(56) Entgegenhaltungen:
**US-A- 5 342 524     US-A- 6 020 185**

• **GONZALEZ-GIL G ET AL: "Effect of yeast extract on speciation and bioavailability of nickel and cobalt in anaerobic bioreactors" BIOTECHNOLOGY AND BIOENGINEERING 20030420 JOHN WILEY AND SONS INC. US, Bd. 82, Nr. 2, 20. April 2003 (2003-04-20), Seiten 134-142, XP002524541**
• **HU ET AL: "Enhancement of methane fermentation in the presence of Ni<2+> chelators" BIOCHEMICAL ENGINEERING JOURNAL, ELSEVIER, AMSTERDAM, NL, Bd. 38, Nr. 1, 7. Dezember 2007 (2007-12-07), Seiten 98-104, XP022382892 ISSN: 1369-703X**
• **CALLANDER I J ET AL: "PRECIPITATION, CHELATION, AND THE AVAILABILITY OF METALS AS NUTRIENTS IN ANAEROBIC DIGESTION. II. APPLICATIONS." BIOTECHNOLOGY AND BIOENGINEERING 1983 AUG, Bd. 25, Nr. 8, August 1983 (1983-08), Seiten 1959-1972, XP002524831**
• **CALLANDER I J ET AL: "PRECIPITATION, CHELATION, AND THE AVAILABILITY OF METALS AS NUTRIENTS IN ANAEROBIC DIGESTION. I. METHODOLOGY." BIOTECHNOLOGY AND BIOENGINEERING 1983 AUG, Bd. 25, Nr. 8, August 1983 (1983-08), Seiten 1947-1957, XP002524832**
• **G. BERTHON: "The stability constants of metal complexes of amino acids with polar side chains" PURE AND APPLIED CHEMISTRY, Bd. 67, Nr. 7, 1995, Seiten 1117-1240, XP002524833**
• **J. RINTALA ET AL: 'Thermophilic anaerobic treatment of sulfate-rich pulp and paper integrate process water' WAT. SCI. TECH. 01 Januar 1991, Seiten 149 - 160, XP055003377**

EP 2 231 528 B1

**Beschreibung**

Technisches Gebiet:

Die Erfindung betrifft Zusatzstoffe für anaerobe Fermentationen, insbesondere Verfahren zur Gewinnung von Biogas, die die Verfügbarkeit von Spurenelementen für die Mikroorganismen verbessern.

Stand der Technik:

Biogas ist ein Gemisch aus den Hauptkomponenten Methan und $CO_2$. Daneben enthält es geringe Mengen an Wasserdampf, $H_2S$, $NH_3$, $H_2$, $N_2$ und Spuren von niedrigen Fettsäuren und Alkoholen.

In einer Biogasanlage wird Substrat unter Sauerstoffabschluss zu Biogas ($CO_2$ und $CH_4$) vergoren. Diese Vergärung wird in vier Teilschritte unterteilt: Die fermentative Phase, in der große Biopolymere gelöst werden, die acidogene Phase, in der die gelösten Mono- und Oligomere zu organischen Säuren, Alkoholen, $CO_2$ und Wasserstoff umgesetzt werden, die acetogene Phase, in der die organischen Säuren und Alkohole zu Essigsäure, Wasserstoff und $CO_2$ umgewandelt werden und schließlich die methanogene Phase, in der aus Essigsäure oder $CO_2$ und Wasserstoff Methan gebildet wird. Darüber hinaus werden im Biogasverfahren noch die reduzierten, teilweise wasserlöslichen Endprodukte $NH_3$ und $H_2S$ gebildet.

Die hierfür benötigten Mikroorganismen katalysieren die notwendigen Umsetzungsreaktionen durch Enzyme. Viele Enzyme, insbesondere die Enzyme, die für die Regulation des Reduktionsäquivalente-Haushalts verantwortlich sind, benötigen Metallionen als Coenzym.

Als Beispiel können die Hydrogenasen (EC 1.12.x.x) angegeben werden. Hydrogenasen katalysieren die Reaktion:

$$2H^+ + Elektronendonor \leftrightarrow H_2 + Elektronenakzeptor$$

Diese sind somit an dem im Biogasverfahren sehr wichtigen Schritt der Wasserstoffproduktion beteiligt. Neben Cosubstraten wie FAD(H), NAD(P)(H) oder Ferredoxin, die auch Spurenelemente (z.B. Fe) enthalten können, benötigen diese Enzyme die Cofaktoren Ni (z.B. EC1.12.1.2), Fe-S Verbindungen (z.B. EC1.12.5.1) oder Se (z.B. EC1.12.2.1).

Ein weiteres wichtiges Enzym bei der Methansynthese, welches Spurenelemente (insbesondere Co) benötigt, ist die Acetyl-CoA:Corrinoid Protein O-Acetyltransferase (EC2.3.1.169), welche Acetyl-CoA zu einer Methylgruppe, Kohlenstoffmonoxid und CoA reagieren lässt, z.B. in *Methanosarcina barkeri.*

Die Versorgung der Mikroorganismen im Biogasverfahren mit den essentiellen Spurenelementen (Mikronährstoffen) wird durch die Gegenwart von $H_2S$, welches zu 2H+ + $S^{2-}$ dissoziiert unterbunden. Viele der wichtigen Spurenelemente bilden schwerlösliche Sulfide, sobald auch nur geringe Mengen an $H_2S$ in Lösung sind. Beispielsweise sind bei folgenden Annahmen: pH7, 37°C, 500ppm $H_2S$ im Biogas, m(S) >>m(Ni), ideale Mischung, Gleichgewicht zwischen Gas- und Flüssigphase, c(Ni)=5$\mu$mol/L nur $3x10^{-17}$ mol/L, d.h. 0,000.000.001% des Nickels in wässriger Lösung und somit frei und bioverfügbar. Bei Kupfer ist die Sulfidfällung sogar so stark, dass unter den Annahmen wie oben ein 1000 $m^3$-Reaktor rechnerisch nur $10^{-5}$ $Cu^{2+}$-Ionen enthält; das Kupfer ist somit nicht bioverfügbar.

Das Anion der Kohlensäure ($CO_3^{2-}$) bildet besonders mit den Vertretern der Gruppe der seltenen Erden schwerlösliche Verbindungen. Da die Gasphase über einem Anaerobreaktor bis zu 50% $CO_2$ erhalten kann und dazu noch häufig eine Stofftransportlimitation des $CO_2$ aus der Flüssigphase in die Gasphase und ein erhöhter hydrostatischer Druck am Boden von hochbauenden Reaktoren auftritt, spielen die Fällungsreaktionen des Carbonats eine wichtige Rolle bei der Bioverfügbarkeit des $Ca^{2+}$ und $Mg^{2+}$.

In der Offenlegungsschrift DE10300082A1 wird die Zugabe einer Spurenelementlösung zu einem Anaerobreaktor offenbart. Die Spurenelemente werden als Sulfat-, Chlorid-, Selenat- oder Molybdatsalze in wässriger Lösung den Reaktor gegeben, ohne dass auf die Bioverfügbarkeit der Spurenelemente Rücksicht genommen wird. In Anwesenheit von $H_2S$ wird ein Großteil (>99,9%, siehe oben) der fällbaren Ionen als Sulfide ausfallen. Die Art des Anions des Spurenelement-Salzes ist für die Bioverfügbarkeit des jeweiligen Spurenelements nicht von Bedeutung.

Handelsübliche Spurenelementzusammensetzungen, die als Zuschlag für Substrate, insbesondere von pflanzlichen Agrarrohstoffen oder Industrieabwässern, Verwendung finden werden in einer Menge von ca. 1-2 kg/Tonne Trockensubstanz des Substrats eingesetzt. Wegen der starken Sulfid-Ausfällung der Metalle während der Fermentation, kann der Fermentationsrückstand nicht als Düngemittel verwertet werden, da die erlaubten Metallkonzentrationen für Düngemittel weit überschritten werden.

Um die Löslichkeit der Spurenelemente zu erhöhen können die Spurenelemente in saurer Lösung angesetzt werden. Durch den niedrigeren pH-Wert wird das Dissoziationsgleichgewicht von $H_2S$ und $S^{2-}$ zu $H_2S$ verschoben und somit der Ausfällung vorgebeugt. Auch die Ausfällung von schwerlöslichen Hydroxidsalzen wird so verhindert. Nach der Einbringung in den Biogasreaktor werden die so gelösten Spurenelemente jedoch wieder als Sulfide ausfallen, da beispielsweise in einem Biogasreaktor ein pH-Wert von 6-8 vorliegt.

Eine weitere Möglichkeit Spurenelemente bioverfügbar zu machen, ist deren Immobilisierung auf organischen Trägermaterialien (DE10139829A1), Getreideextrudaten (DE10226795A1) Mineralischen Formkörpern (EP0328560B1) oder Zeolithen (AT413209B). Diese Form der Darreichung hat den Vorteil, dass sich die Mikroorganismen auf den Trägern ansiedeln und die benötigten Spurenelemente aus den Trägern in die Mikroorganismen diffundieren können ohne dabei gefällt zu werden. Als Nachteil dieser Methode ist anzuführen, dass dies nur bei niedrig konzentrierten Feststoffsuspensionen und niedrigen Viskositäten möglich ist. In einem Bioreaktor mit hohen Feststoffkonzentrationen bei dem Stofftransportphänomene eine wichtige Rolle spielen, können die Mikroorganismen so nicht versorgt werden. Außerdem neigen anaerobe Kulturen dazu, sehr stabile Biofilme zu bilden, die im Laufe der Zeit einen Transportwiderstand darstellen würden. Ebenso ist zu erwähnen, dass einige anaerobe Bakterien (z.B. Cellulose-abbauende Clostridien) sich direkt auf dem Substrat auf den Trägermaterialien niederlassen müssen, um dies zu verdauen. Eine zusätzliche Versorgung dieser Zellen mit Spurenelementen auf fixierten Trägern ist somit kaum möglich.

Die Wirkungsgrade dieser Dosierungsmethoden sind jedoch niedrig, d.h. nur ein Bruchteil der zudosierten Spurenelemente wird tatsächlich bei der Biogasgewinnung biologisch verwertet. Der überwiegende Teil der Spurenelemente fällt als Sulfid in den Klärschlamm aus oder verbleibt in stark komplexierter Form im flüssigen Gärrückstand. Die festen und flüssigen Gärrückstände sollen als Dünger auf die Felder aufgebracht werden, auf denen die zukünftigen Substrate für die Biogasanlage heranwachsen. Ein dauerhaftes Supplementieren des Biogasreaktors mit großen Mengen von Spurenelementen würde zu einer Anreicherung der in hohen Konzentrationen giftigen, Spurenelemente führen. Eine Verbesserung der Darreichungsform der Spurenelemente würde die benötigte Menge der Spurenelemente und somit auch die Schwermetallbelastung des Gärrückstands reduzieren.

Aus der US 5,342,524 ist bekannt, dass mit der Zugabe von bestimmten Komplexbildnern zu einem Substrat für eine anaerobe Biogasfermentation, die Löslichkeit der Spurenelemente in der Fermentationsbrühe erhöht und dadurch die Methanausbeute wesentlich verbessert werden kann.

Gonzalez-Gil G. et al. (Biotechnology and Bioengineering, 2003, 82(2):134-142) beschreibt die komplexierende Wirkung von Hefeextrakt bzgl. $Ni^{2+}$ und $Co^{2+}$. Die Spurenelemente ($Ni^{2+}$ und $Co^{2+}$) werden in Form von Chloridsalzen in sauren und alkalischen Lösungen den Ansätzen zudosiert. Der Komplexbildner, Hefeextrakt, wird separat zu den Spurenelementen in die Gefäße beigemischt.

Hu et al. (Biochemical Engineering, 2007, 38(1):98-104) beschreibt die Verbesserng der Methangärung in Gegenwart der Nickel-Chelatbildner Zitronensäure, NTA und EDTA. Es wird berichtet, dass die Zugabe eines Komplexbildners (CA, NTA oder EDTA) zur Bildung löslicher Komplexe führt und dadurch die Spurenelemente aus den ausgefallenen Sulfiden herauslöst, wodurch die Bioverfügbarkeit von Nickel erhöht wird.

Callander IJ et al. (Biotechnology and Bioengineering, 1983, 25(8):1959-1972) beschreibt Methoden zur Ermittlung der Konzentrationen an frei verfügbaren Metallen bei Biogasfermentationen anhand von zwei Fallstudien; einem definierten Medium und einem komplexen Medium.

Rintala J. et al. (Water Science and Technology, 1991, 24(3/4):149-160) beschreibt Fermentationen mit Abwässern aus der Papierherstellung, wobei Sulfatreiche Abwässer mit verschiedenen Fermentern und Impfschlämmen abgebaut werden. Es wird eine Nährstofflösung mit verschiedenen Mikro- und Makronährstoffen eingesetzt, die auch Spurenelemente enthält

## Beschreibung der Erfindung

Die Aufgabe der Erfindung liegt darin, Spurenelemente für eine anaerobe Fermentation, insbesondere für ein Biogasverfahren, in einer verbesserten Formulierung bereitzustellen, die gegenüber Störstoffen wie Fe(III), und, gegebenenfalls Stoßbelastungen stabil ist und die Bioverfügbarkeit der Spurenelemente und somit deren Umsetzung durch die im Bioreaktor vorhandenen Mikroorganismen erhöht; wobei im Fermentationsrückstand die erlaubten Grenzwerte der Schwermetallkonzentrationen in Düngemittel nicht überschritten werden sollen. Die Aufgabe wird durch die in den Patentansprüchen definierten Gegenstände gelöst.

Unter "Bioverfügbarkeit" wird der Anteil bzw. die Darreichungsform eines Spurenelements verstanden, welche von den Mikroorganismen im Bioreaktor resorbiert werden kann. Vorzugsweise handelt es sich dabei um eine unter den Fermentationsbedingungen lösliche, d.h. nicht ausgefallene, Form bzw. Verbindung des Spurenelements.

Die Erfindung betrifft eine Spurenelementlösung zum Supplementieren von Spurenelementen in anaeroben Fermentationen, insbesondere für Verfahren zur Herstellung von Biogas, die unter neutralen oder schwach sauren Milieubedingungen durchgeführt werden, bei denen Spurenelemente, beispielsweise, als Sulfidsalze ausfallen können.

Neben zumindest einem, bevorzugt mehreren, Spurenelementen umfasst die Lösung Komplexbildner. Komplexbildner sind Verbindungen, die zur Komplexierung und Maskierung von Metallen geeignet sind. Einige sind auch unter der Bezeichnung "Chelatbildner" bekannt. Die Komplexbildung entsteht durch eine koordinative Bindung zwischen dem Metallatom und einem oder mehreren Molekülen, d.h. Liganden, des Komplexbildners, die das Metallatom umschließen. Die Komplex-Bildungskonstanten der erfindungsgemäßen Komplexbildner müssen hoch genug sein, um unter Berücksichtigung des pH-Wertes sowie der Dissoziationskonstanten der Komplexbildner und des $H_2S$ die jeweiligen Spurenelemente der erfindungsgemäßen Lösung in Gegenwart der Sulfid-Ionen im Fermenter in Lösung zu halten.

Ein Spurenelement fällt nicht mit einem entsprechenden, gegenwärtigen, Anion (z.B. $S^{2-}$, $CO_3^{2-}$ oder $OH^-$) aus, wenn folgende Bedingung erfüllt ist:

$$K_L\left(\frac{H^+ + K_a}{K_a} - 1\right) \geq \frac{K_{SP}}{A}$$

$K_L$      Stabilitätskontante des Komplexes
$H^+$      $H^+$-Konzentration
$K_a$      Dissoziationskonstante des Komplexbildners
$K_{SP}$      Löslichkeitsprodukt
$A$      Anionenkonzentration ($S^{2-}$, $CO_3^{2-}$, $OH^-$) =f(pH), steigt mit steigendem pH

Die Lösung umfasst Komplexbildner und Spurenelemente in zumindest äquimolarer Menge, so dass die meisten zugefügten Spurenelemente im Fermenter weitgehend als Komplexe vorliegen. Gegebenenfalls, können die erfindungsgemäßen Komplexbildner im Überschuss in der Spurenelementelösung enthalten sein. Der Überschuss an erfindungsgemäßen Komplexbildnern kann ein Vielfaches der Spurenelementelösung betragen, um auch aus dem Substrat austretende (z.B. $Mg^{2+}$, $Ca^{2+}$) oder in den Bioreaktor zugeführte (z.B. $Fe^{3+}$) Metallionen zu komplexieren. Die Erfinder haben gefunden, dass in einem beispielhaften Referenzsystem aus Wasser-EDTA-$Fe^{2+}$-$Ni^{2+}$-$Co^{2+}$-$H_2S$ ein starker Komplexbildner wie EDTA (Ethylendiamintetraessigsäure) in äquimolaren Mengen Spurenelemente komplexiert, obwohl bei neutralen pH-Werten nur ein kleiner Teil des EDTA in einer Wasser-EDTA-Mischung als aktives $EDTA^{4-}$-Anion vorliegt. Auch die Anwesenheit von $H_2S$ verursacht in Gegenwart von EDTA keine Ausfällungen. Werden statt EDTA Komplexbildner verwendet, die Komplexe aus zwei oder mehreren Liganden der Komplex-bildner pro Metallatom ausbilden, muss eine entsprechend vielfache (zwei-, mehrfache) molare Menge der entsprechenden Liganden eingesetzt werden, um die Spurenelemente in der Lösung zu komplexieren.

Wird nun die Menge des Komplexbildners (z.B. EDTA) schrittweise reduziert, so fallen in der Reihenfolge der Komplex-Bildungskonstanten (pK) zuerst $Fe^{2+}$ (pK=14,3), dann $Co^{2+}$ (pK=16,3), und schließlich $Ni^{2+}$-Ionen (pK=18,6) aus. Das gleiche geschieht, wenn ein Störstoff zugegeben wird (wie z.B. $Fe^{3+}$, pK=25,1; $Mn^{3+}$; $Hg^{2+}$), der stärkere Bindungen mit dem Komplexbildner eingeht und Metalle mit niedrigeren Komplex-Bildungskonstanten verdrängt. Ein Ziel der Erfindung ist es, die erfindungsgemäße Spurenelementlösung so zu formulieren, dass auch bei derartigen Störungen von verschiedenen Metallionenspezien (z.B. $Fe^{3+}$, $Mg^{2+}$) noch eine ausreichende Menge Ionen der anderen Metallionen der Spurenelementelösung komplexiert in Lösung bleiben, um eine Limitation dieser Ionen, insbesondere $Co^{2+}$, $Ni^{2+}$ oder $Mn^{2+}$, während der Fermentation auszuschließen.

Durch teilweisen Austausch des starken Komplexbildners wie z.B. EDTA mit einem Gemisch aus zwei verschiedenen Komplexbildnern mit unterschiedlichen Affinitäten, d.h. Komplex-Bildungskonstanten, zu den Metallen können die meisten Spurenelemente vollständig unter den Bedingungen einer Biogasfermentation komplexiert werden und stehen außerdem dem System selbst bei einer Störung durch eine Metallspezies wie $Fe^{3+}$, $Mg^{2+}$ oder $Ca^{2+}$, immer noch teilweise komplexierte Spurenelemente zur Verfügung. Nur ein Teil der jeweiligen Metallspezies wird dann durch das Sulfid in der Fermentationsbrühe gefällt. Die Lösung mit Spurenelementen, umfasst zumindest zwei verschiedene Komplexbildner , wobei die Komplexbildner sich in den Komplex-Bildungskonstanten bzw. Affinitäten zu Metallionen unterscheiden. D.h. es werden, verschiedene Komplexbildner ausgewählt, die die Metallionen unterschiedlich stark komplexieren, wobei sie ausreichend stark sein sollen, um ein Ausfällen unter den Bedingungen einer Biogasfermentation zu verhindern. Gegebenenfalls können auch drei, vier, fünf oder mehr Komplexbildner in der erfindungsgemäßen Lösung enthalten sein. Durch die Verwendung von zwei oder mehr verschiedenen Komplexbildnern wird der Wirkungsgrad der Spurenelementdarreichung erhöht und eine Darreichungsform für die Spurenelemente erhalten, die auch bei schwankenden Reaktionsbedingungen stabil bleibt. Denn wird eine Metallspezies aus einem Komplex durch eine andere Metallspezies verdrängt, die eine größere Affinität (pK) zu diesem Komplexbildner besitzt, wird die verdrängte Metallspezies dann einen neuen Komplex mit einem zweiten Komplexbildner ausbilden. Die Verwendung von zumindest zwei verschiedenen Komplexbildnern ermöglicht auch, dass schwerlösliche Mikronährstoffe wie Kobalt, Nickel oder Mangan trotz der hohen Belastung mit Sulfid- und Carbonationen bei einer Biogasfermantation vermehrt zur Verfügung stehen

Außerdem wird durch die Verwendung der zumindest zwei verschiedenen Komplexbildnern gemäß der Erfindung die Bioverfügbarkeit insbesondere von Kobalt, Nickel, Zink und Mangan wesentlich erhöht und die Ausbeute des Biogasverfahrens stark verbessert, da insbesondere Kobalt und Nickel essentiell für die Methanogenese sind. Besonders vorteilhaft wird mit der erfindungsgemäßen Spurenelementlösung die Bioverfügbarkeit von Kobalt um ein Vielfaches erhöht. Gleichzeitig wird durch die Erhöhung der Bioverfügbarkeit und damit der Löslichkeit, die notwendige Menge an Spurenelemente für eine entsprechende Effizienzsteigerung des Verfahrens stark vermindert. So kann bereits die Zugabe einer erfindungsgemäßen Spurenelementelösung von, beispielsweise, 30 mL/Tonne Trockensubstanz des Fermentationssubstrats für die Supplementierung, insbesondere eines Monosubstrats, ausreichend sein.

Tabelle 1 zeigt, wie bei gleicher Einsatzmenge an Spurenelementen deren Konzentration bzw. Bioverfügbarkeit verbessert wird: Wenn die Spurenelemente mit dem Chelat-Komplexbildner NTA komplexiert werden, wobei NTA in einer stöchiometrischen Menge von 50% der gesamten Menge der Spurenelemente einer Spurenelementelösung zugesetzt wird (wie in der Beschreibung der bekannten Spurenelementelösung für das Medium 141 der DSMZ (Deutsche Sammlung für Mikroorganismen und Zellkulturen); siehe Tabelle 5), sind Kobalt, Nickel und Zink kaum bioverfügbar. Nur 2 ppm der zugefügte Nickelmenge sind verfügbar. Auch durch die alleinige Verwendung einer überstöchiometrischen Menge von 500% Citrat (5-fache stöchiometrische Menge gegenüber der Spurenelementelösung) wird die Bioverfügbarkeit von essentiellen Spurenelementen wie Kobalt oder Nickel nicht verbessert. Andererseits, wenn eine Spurenelementelösung mit zwei Komplexbildnern verwendet wird, nämlich EDTA und einer Mischung aus Phosphorsäuren, sind Kobalt, Nickel und Mangan zu 100% bioverfügbar. In der Tabelle 1 enthält die Spurenelementelösung die Komplexbildner in einer stöchiometrischen Menge von 60% EDTA und 60% einer Phosphorsäurenmischung gegenüber der Gesamtmenge der Spurenelemente, die aus gleichen molaren Mengen von Pyrophosphorige Säure ($H_4P_2O_7$), Poly-Phosphorige Säure ($H_6P_4O_{13}$), Meta-Phosphorige Säure ($H_4P_4O_{12}$), Hypo-Phosphorige Säure ($H_3PO_2$) und Phosphorige Säure (Phosphonsäure) ($H_3PO_3$) zusammengesetzt ist.

Da durch die Verwendung von zwei oder mehr verschiedenen Komplexbildnern die Bioverfügbarkeit von Kobalt, Nickel und Zink derart erhöht wird, kann Einsatzmenge der Metalle in der Spurenelementelösung deutlich verringert werden.

Tabelle 1: Konzentrationen der Spurenelemente in mol/L

|  | Lösung, ohne Komplex | Gärrest ohne Komplex | Gärrest mit 50% NTA[1] | Gärrest Citrat (500%)[2] | BioverfügBarkeit | Gärrest 60% EDTA 60% Phosphorsäuren[3] | Bioverfügbarkeit |
|---|---|---|---|---|---|---|---|
| Fe | 2,0E-06 | 4,3E-10 | 2,0E-06 | 2,0E-06 | 100% | 2,0E-06 | 100% |
| Mg | 1,4E-04 | 1,4E-04 | 1,4E-04 | 1,4E-04 | 100% | 1,4E-04 | 100% |
| Ca | 5,0E-06 | 5,0E-06 | 5,0E-06 | 5,0E-06 | 100% | 5,0E-06 | 100% |
| Cu | 2,2E-07 | 6,9E-37 | 8,2E-30 | 5,1E-22 | 0% | 2,5E-22 | 0% |
| Co | 3,4E-06 | 6,9E-15 | 8,2E-11 | 5,1E-08 | 0% | 3,4E-06 | 100% |
| Ni | 4,7E-07 | 8,7E-19 | 1,0E-12 | 6,4E-12 | 0% | 4,7E-07 | 100% |
| Zn | 3,1E-06 | 9,5E-17 | 1,1E-11 | 7,0E-10 | 0% | 3,1E-06 | 100% |
| Mn | 2,3E-05 | 6,1E-08 | 7,2E-06 | 4,5E-06 | 31% | 2,3E-05 | 100% |
| Al | 3,2E-07 | 3,2E-07 | 3,2E-07 | 3,2E-07 | 100% | 3,2E-07 | 100% |
| [1] 0,5-fach stöchiometrische Menge NTA<br>[2] 5-fach stöchiometrische Menge Citrat<br>[3] 0,6-fach stöchiometrische Menge EDTA und 0,6-fach stöchiometrische Menge der Phosphorsäurenmischung | | | | | | | |

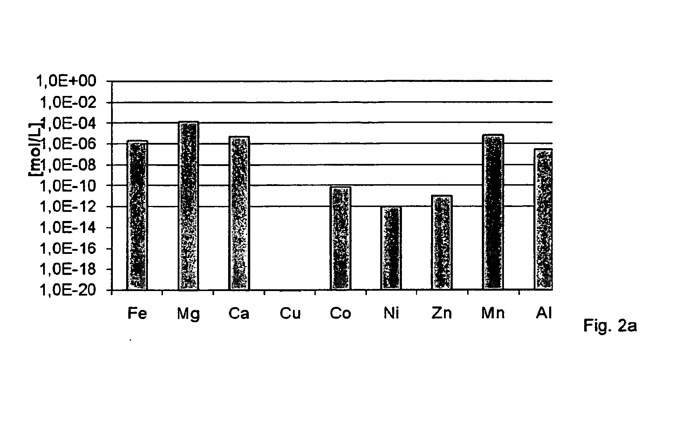

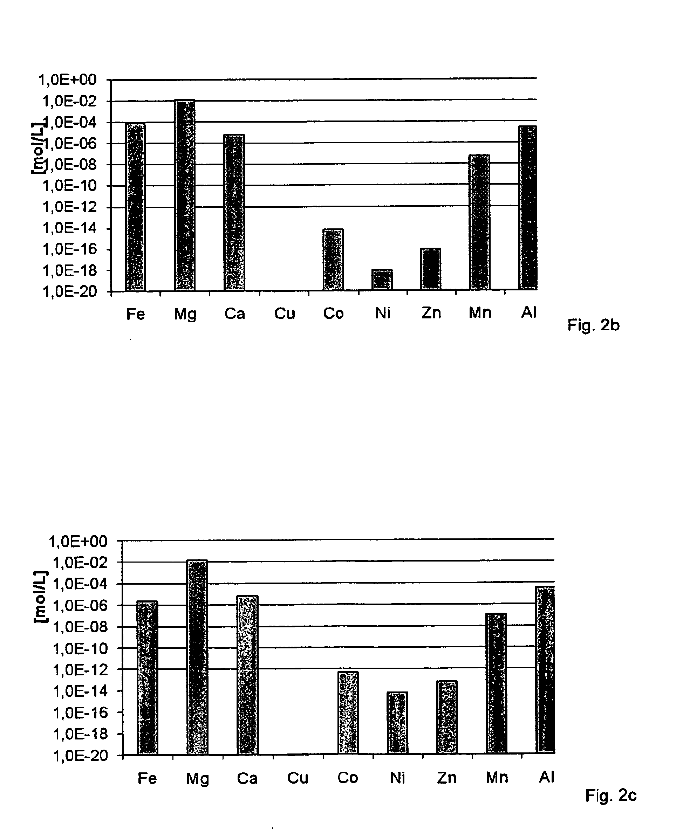

Tabelle 2, linke Spalte, zeigt, dass die Bioverfügbarkeit der Spurenelemente nicht verbessert werden kann, wenn deren Konzentration in der Spurenelementelösung mit einem Komplexbildner (NTA) 100-fach erhöht wird. Im Gegenteil, der bioverfügbare Anteil von Kobalt, Nickel und Mangan nimmt ab. Kobalt, Nickel und Mangan werden nämlich durch Eisen, dessen Konzentration ebenfalls zunimmt, aus den Komplexen verdrängt. (Kobalt, Nickel und Mangan haben eine niedrigere Komplexbildungskonstante als Eisen). Es ist dann kein Komplexbildner mehr übrig, der Nickel, Kobalt und Mangan komplexieren kann. Das ist in den Figuren 2a und 2b dargestellt.

Auch wenn in einer Spurenelementelöung mit einem Komplexbildner (NTA) der Eisen Anteil konstant gehalten wird und nur die Konzentration der anderen Spurenelemente 100-fach erhöht wird, sinkt der bioverfügbare Anteil von Kobalt, Nickel und Mangan, obwohl diese Spurenelemente eine größere Komplexbildungskonstante als Kalzium oder Magnesium haben. Das ist in der Tabelle 2, rechte Spalte und Figur 2c dargestellt. Kobalt, Nickel und Mangan sind nämlich weniger löslich als Kalzium oder Magnesium. Da die Konzentration des freien $Mg^{2+}$ etwa 17 Größenordnungen höher ist, als die Konzentration des freien $Ni^{2+}$, wird der Komplex zwischen NTA und Magnesium gebildet, obwohl die Komplexbildungskonstante für Ni mit pK=12 wesentlich höher ist, als die für Magnesium mit pK= 6. Dieses Phänomen tritt besonders bei anaeroben Biogasfermentationen auf, da hier große Konzentrationen an $CO_3^{2-}$ und $S^{2-}$ entstehen. Mit einer erfindungsgemäßen Spurenelementelösung kann hingegen die Bioverfügbarkeit von Kobalt, Nickel und Mangan wesentlich verbessert werden und diese Metalle auch bei einer großen Belastung durch $CO_3^{2-}$ und $S^{2-}$ ausreichend in Lösung gehalten werden.

Tabelle 2: Konzentrationen der Spurenelemente in mol/L

| | Gärrest mit 50% NTA[1] | | | Gärrest mit 50% NTA[1] 100-fach Metall[4] 1-fach Fe |
|---|---|---|---|---|
| | Einfach[2] | 100-flach Metall | Unterschied | |
| Fe | 2,00E-06 | 8,72E-05 | 4260,1% | 2,00E-06 |
| Mg | 1,40E-04 | 1,37E-02 | 9698,1% | 1,37E-02 |
| Ca | 5,00E-06 | 6,91 E-06 | 38,2% | 6,95E-06 |
| Cu | 8,18E-30 | 6,94E-37 | -100,0% | 4,28E-32 |
| Co | 8,18E-11 | 6,94E-15 | -100,0% | 4,35E-13 |
| Ni | 1,02E-12 | 8,68E-19 | -100,0% | 5,36E-15 |
| Zn | 1,12E-11 | 9,55E-17 | -100,0% | 5,90E-14 |
| Mn | 7,22E-06 | 6,07E-08 | -99,2% | 9,82E-08 |
| Al | 3,20E-07 | 3,21 E-05 | 9931,3% | 3,21 E-05 |
| [1] Spurenelemente mit 0,5-fach stöchiometrischer Menge NTA wie in Tabelle 1 komplexiert [2] Spurenelementezusammensetzung wie in Tabelle 5 [3] 100-fache Menge der Spurenelementemenge von Tabelle 5 [4] 100-fache Menge von Mg, Ca, Cu, Co, Ni, Zn, Mn, Al und 1-fache Menge Fe von der Menge in Tabelle 5 | | | | |

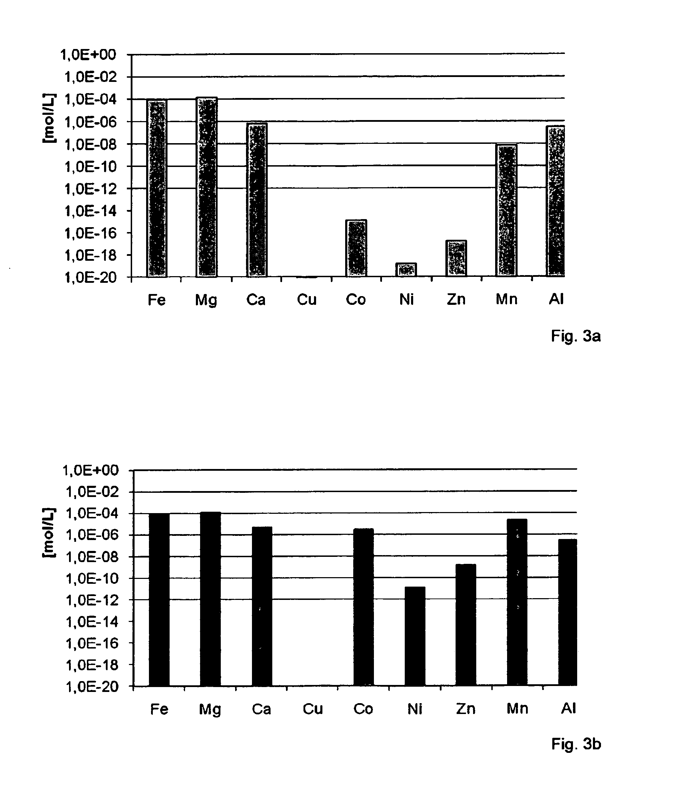

In Tabelle 3 und Figur 3 ist dargestellt, wie Störstoffe (Fe(III)) die Konzentration und Bioverfügbarkeit der Spurenelemente in einer Lösung mit nur einem Komplexbildner beeinflussen. Sobald größere Mengen Fe(III) aus dem Fermentationssubstrat in die Fermentationsbrühe gelangen kommt es bei einer Spurenelemntelösung, die nur einen Komplexbildner umfasst, zu Ausfällungen der anderen Spurenelemente, da Fe(III) eine größere Affinität zu dem Komplexbildner hat und die anderen Spurenelemente aus den Komplexen verdrängt, worauf das Spurenelement ausfällt. Wird hingegen eine Spurenelementelösung gemäß der Erfindung mit zumindest zwei verschiedenen Komplexbildnern verwendet, so bleibt die Bioverfügbarkeit der Spurenelemente für die Biogasfermentation aufrecht. Der Tabelle 3 und Figur 3b ist zu entnehmen, im welchen Ausmaß in diesem Beispiel die Bioverfügbarkeit der Spurenelemente erfindungsgemäß selbst bei Zugabe eines Störstoffes (Fe(III)) gegenüber Lösungen verbessert wird, die nur einen Komplexbildner (NTA) enthalten.

Tabelle 3: Konzentrationen der Spurenelemente im Gärrest in mol/L

| | 50% NTA[1) kein Fe (III) | 50% NTA[1) 500% Fe(III)[2) | Erfindung | |
|---|---|---|---|---|
| | | | 60%EDTA+60%P-Mix[3); 500%Fe(III) | 100%EDTA+1000%P-Mix[4), 500%Fe(III) |
| Fe | 2,0E-06 | 8,9E-05 | 1,0E-04 | 1,0E-04 |
| Mg | 1,4E-04 | 1,4E-04 | 1,4E-04 | 1,4E-04 |

(fortgesetzt)

| | 50% NTA[1] kein Fe (III) | 50% NTA[1] 500% Fe(III)[2] | Erfindung | |
|---|---|---|---|---|
| | | | 60%EDTA+60%P-Mix[3]; 500%Fe(III) | 100%EDTA+1000%P-Mix[4], 500%Fe(III) |
| Ca | 5,0E-06 | 6,4E-07 | 5,0E-06 | 5,0E-06 |
| Cu | 8,2E-30 | 1,2E-37 | 3,1E-36 | 1,1E-21 |
| Co | 8,2E-11 | 1,2E-15 | 3,4E-06 | 3,4E-06 |
| Ni | 1,0E-12 | 1,5E-19 | 1,1E-18 | 1,4E-11 |
| Zn | 1,1E-11 | 1,7E-17 | 9,6E-17 | 1,5E-09 |
| Mn | 7,2E-06 | 7,3E-09 | 2,5E-07 | 2,3E-05 |
| Al | 3,2E-07 | 3,2E-07 | 3,2E-07 | 3,2E-07 |

[1] 0,5-fach stöchiometrische Menge NTA(50% der molaren Gesamtmenge Spurenelemente)

[2] 0,5-fach stöchiometrische Menge NTA(50%), Zugabe von 5-fach stöchiometrischer Menge (500%) Fe (III) (500% der molaren Gesamtmenge der Spurenelemente)

[3] 0,6-fach stöchiometrische Menge EDTA (60%) und 0,6-fach stöchiometrische Menge Mischung phosphorige Säuren (=60% P-Mix) der Phosphorsäurenmischung von Tabelle 1

[4] einfach stöchiometrische Menge EDTA (100%) und zehnfach stöchiometrische Menge Mischung phosphorige Säuren (=1000% P-Mix) der Phosphorsäurenmischung von Tabelle 1 bezogen auf die Gesamtmenge Spurenelemente

Starke Komplexbildner wie zum Beispiel EDTA oder NTA können alle Spurenelemente einer Spurenelementelösung mit der Ausnahme von Kupfer vollständig komplexieren. Wird jedoch nur ein Komplexbildner in der Spurenelementelösung verwendet, führt die Zugabe von Fe(III) zur Umkomplexierung; z.B. FeCl$_3$ zur Entschwefelung des Bioreaktors oder in pflanzlichen Substraten gebundenes Fe(III). Dabei löst das Fe(III) das EDTA vom Spurenelement und liegt dann als Fe-EDTA komplexiert vor. Das Spurenelement fällt aus. Ein ähnliches Pänomen tritt mit Kalzium und Magnesium aufgrund der geringen Löslichkeit der essentiellen Spurenelemente Kobalt, Nickel und Mangan ein, wobei dann die Makroelemente Kalzium und Magnesium die Mikroelemente Kobalt, Nickel und Mangan aus den Komplexen verdrängen.

Somit ist eine Ausführungsform der Erfindung eine Spurenelementelösung mit zumindest zwei Komplexbildnern, die sich in der Komplexbildungskonstante (pK) für Fe$^{3+}$ unterscheiden. Fe$^{3+}$ wird dann mit dem Komplexbildner komplexiert, zu dem es eine höhere Affinität (pK) hat. Der zumindest eine, andere Komplexbildner steht dann zur Komplexierung der anderen Spurenelemente zur Verfügung. Es werden deshalb die Komplexbildner so gewählt, dass zumindest ein erster Komplexbildner Fe$^{3+}$ stabil komplexieren kann, und zumindest ein zweiter Komplexbildner die anderen Spurenelemente unter den Bedingungen (pH-Wert, [S$^{2-}$], [CO$_3^-$]) einer Biogasfermentation komplexieren kann; auch in Gegenwart von Fermentationssubstraten, die reich an Ca$^{2+}$ und/oder Mg$^{2+}$ sind. Die erfindungsgemäße Spurenelementlösung verbessert auch die Bioverfügbarkeit der Spurenelemente bei anderen anaeroben und aeroben Fermentationen, insbesondere bei Verfahren mit Bedingungen bei denen Spurenelemente ausfallen können.

Bevorzugt umfasst deshalb die erfindungsgemäße Spurenelementelösung einen ersten Komplexbildner mit einer größeren Komplexbildungskonstante (pK) für Fe$^{3+}$, als für andere Spurenelemente, insbesondere Co$^{2+}$ oder Ni$^{2+}$, und einen zweiten Komplexbildner mit Affinitäten bzw. Komplexbildungskonstanten für Spurenelemente, die ausreichend sind, um die Spurenelemente unter den Bedingungen der Biogasfermentation so stark zu komplexieren, dass sie ausreichend bioverfügbar sind und, bevorzugt deren Ausfällung weitgehend vermieden wird. Hierfür sollte die Komplexbildungskonstante des zweiten Komplexbildner für das jeweilige Spurenelement, vorteilhaft, zumindest pK=2, bevorzugt pK=5-10 und besonders bevorzugt pK≥10 sein. Selbstverständlich kann als zweiter Komplexbildner auch eine Mischung von zwei, drei oder mehreren Komplexbildnern verwendet werden, wobei jeder Komplexbildner zumindest zu einem der Spurenelemente in der Spurenelementelösung eine Komplexbildungskonstante von pK=2, bevorzugt pK=5-10 und besonders bevorzugt pK≥10 aufweist. Beispielsweise wird als der zweite Komplexbildner ein Komplexbildner gewählt, der eine Komplexbildungskonstante (pK) für Co$^{2+}$ und/oder Ni$^{2+}$ von pK=2, bevorzugt pK=5-10 und besonders bevorzugt pK≥10 aufweist.

Die Komplexbildungskonstante (pK) für Fe$^{3+}$ des zweiten Komplexbildners ist kleiner (schwächer) als die Komplexbildungskonstante (pK) für Fe$^{3+}$ des ersten Komplexbildners. Besonders bevorzugt ist der erste Komplexbildner ein starker Komplexbildner mit einer Komplexbildungskonstante (pK) für Fe$^{3+}$ von mindestens pK=10, bevorzugt pK≥20, besonders bevorzugt pK≥20. Gegebenenfalls können sich die Komplexbildungskonstanten (pK) für Fe$^{3+}$ des ersten und

zweiten Komplexbildners zumindest um das 2-, 3-,4- oder 5-fache von einander unterscheiden.

Die Komplexbildner sind in unterschiedlichen Mengen in der Spurenelementelösung enthalten. Zumindest verbunden ist eine äquimolare Menge der Komplex-bildner gegenüber den Spurenelementen. Vorteilhaft ist auch die Zugabe der Komplexbildner im Überschuss zu den Spurenelementen, beispielsweise 10-, 30-, 50-, 100 oder mehr als 1000-fach, der abhängig ist vom verwendeten Fermentationsubstrat sowie die Fermentationsbedingungen (z.B. Zugabe von $FeCl_3$ für die Entschwefelung). Auch das Mengenverhältnis der verschiedenen Komplexbildner zueinander kann über einen sehr weiten Bereich variieren. Eine schwächeren Komplexbildner bzw. eine Komplexbildnermischung (z.B. Phosphorsäurenmischung) ist in einer vielfachen, z.B. 5-5000 fach, bevorzugt 50-2000fach, besonders bevorzugt, 100-1500fach, molaren Menge eines stärkeren Komplexbildners (z.B. tertiäres Amin) einzusetzen, um die Bioverfügbarkeit der Spurenelemente bzw. die Stabilität der Komplexierung zu optimieren.

Der Begriff Komplexbildungskonstante wird im Rahmen der Anmeldung gleichbedeutend wie Komplexstabilitätskonstante oder Komplexassoziations-konstante verwendet und resultiert aus dem Produkt der individuellen Gleichgewichtskonstanten der Reaktionen bei der Komplexbildung. $K=[ML_n]/[M][L]^n$, wobei $[ML_n]$ die molare Gleichgewichtskonzentration des Metallkomplexes, $[M]$ die molare Gleichgewichtskonzentration des freien Metallions, $[L]$ die molare Gleichgewichtskonzentration des Liganden und n die Anzahl der im Komplex gebundenen Liganden ist. In dieser Anmeldung wird als Wert für die Stabilitätskonstante der pK-Wert angegeben.

Bevorzugt werden Komplexbildner verwendet, die eine Komplexierungskonstante (pK) von zumindest 5, bevorzugt zumindest 10, besonders bevorzugt zumindest 20 für zumindest ein, bevorzugt alle, Metallion(en) der Spurenelementelösung haben und, gegebenenfalls anaerob abbaubar sind.

Die Komplexierungseigenschaften von beispielhaften Komplexbildner mit ausgewählten zwei- und dreiwertigen Metallionen sind in Tabelle 4 angeführt, wobei "+++" für hervorragende (pK>20), "++" für sehr gute (pK=10-20), "+" für gute (pK=5-10), "0" für moderate (pK=2-5), "-" für schlechte (pK=0-2) Komplexierung und "f' für eine Fällung stehen.

**Tabelle 4**

| Komplex-Bildner | Mg++ | Ca++ | Fe++ | Mn++ | Co++ | Ni++ | Cu++ | Al+++ | Fe+++ | Zn++ |
|---|---|---|---|---|---|---|---|---|---|---|
| H4Fe(CN)6 | 0 | 0 | | | | | | | | f |
| Fe(CN)6--- | 0 | 0 | | | | | | | 0 | |
| HCN | | | +++ | | | +++ | | | +++ | f |
| HNCS | | | - | - | - | - | f | | + | |
| NH3 | f | f | | - | + | + | ++ | | | + |
| H3PO2 | | | | | | | | | + | - |
| H3PO3 | | | | | | 0 | f | | + | |
| H3PO4 | f | f | f | | 0 | 0 | 0 | | f | f |
| H4P2O7 | +++ | f | | +++ | + | + | ++ | | | + |
| H5P3O10 | + | + | + | + | + | + | + | | | + |
| H6P4O13 | + | + | | | | | ++ | | | |
| H4P4O12 | + | + | | + | 0 | 0 | + | | | + |
| Cl- | - | - | - | - | - | - | f | | - | f |
| CH2OOH | - | - | | | - | - | 0 | | ++ | - |

(fortgesetzt)

| Komplex-Bildner | Mg++ | Ca++ | Fe++ | Mn++ | Co++ | Ni++ | Cu++ | Al+++ | Fe+++ | Zn++ |
|---|---|---|---|---|---|---|---|---|---|---|
| C2H4OOH | - | - | - | - | - | - | 0 | - | + | - |
| C2H5OOH | - | - | - | - | - | - | 0 | - | 0 | - |
| C3H7OOH | - | - | - | - | - | - | 0 | - | 0 | - |
| (CH3)2CHCOOH | | | | | | | 0 | | 0 | |
| C4H9OOH | | | | | | | 0 | | | |
| (CH3)2C2H3OOH | | | | | | | 0 | | | |
| H2PO3(CH2)2COOH | 0 | 0 | | 0 | 0 | 0 | | | | 0 |
| C3H7O7P | 0 | 0 | | 0 | 0 | 0 | | | | 0 |
| CH2(OH)COOH | - | - | - | - | 0 | 0 | 0 | | 0 | 0 |
| CH3CH(OH)COOH | - | - | | - | 0 | 0 | 0 | | | 0 |
| CH3CH2CH(OH)COOH | | | | | - | 0 | 0 | | | 0 |
| C6H10O7 | | | ++ | | | | | | | |
| C2H2O3 | | | | | | - | | | | - |
| CH3(C=O)COOH | | - | | - | | - | 0 | | | |
| CH2(SH)COOH | | | ++ | + | ++ | +++ | | | | +++ |
| CH3CH(SH)COOH | | | | | | +++ | | | | +++ |
| C5H4O4N2 | | | | | + | + | + | | | |
| (COOH)2 | 0 | f | + | 0 | + | + | + | | ++ | + |
| HOOCCH2COOH | 0 | 0 | | | + | 0 | + | ++ | + | + |
| HOOC(CH2)2COOH | 0 | - | - | 0 | 0 | 0 | 0 | | + | 0 |
| HOOCCH(OH)COOH | 0 | 0 | | | 0 | 0 | + | | | 0 |
| HOOCCH2CH(OH)COOH | - | - | 0 | 0 | 0 | 0 | + | | ++ | 0 |
| HOOCCH(OH)CH(OH)COOH | - | 0 | 0 | 0 | 0 | 0 | + | + | ++ | 0 |
| C6H10O8 | | | | | | | | | +++ | |
| HOOCCH2CH(SH)COOH | | | | | + | ++ | | | 0 | ++ |
| HOOCCH2-S-CH2COOH | | - | + | - | + | + | ++ | ++ | 0 | + |
| HOOCCH2CH(COOH)CH(OH)COOH | 0 | 0 | | 0 | | | | | | |
| HOOCCH2C(OH)(COOH)CH2COOH | 0 | 0 | 0 | 0 | + | + | ++ | | ++ | + |

| | | | | | | | | | | |
|---|---|---|---|---|---|---|---|---|---|---|
| (C6H4)(OH)(COOH) | | | | ++ | ++ | ++ | ++ | | +++ | + |
| CH3(C=O)CH2(C=O)CH3 | + | | + | + | + | ++ | ++ | +++ | +++ | |
| C2H5O2N | 0 | - | + | | | ++ | ++ | | +++ | ++ |
| C3H7O2N | | | | | | ++ | ++ | | | ++ |
| C5H11O2N | | | | | | ++ | ++ | | | |
| C6H13O2N | | | | 0 | | | ++ | | | ++ |
| C9H11O2N | | | | | | | ++ | | | ++ |
| C3H7O2N | | | | | + | | | | | |
| C4H7O4N | | | | | | ++ | ++ | 0 | | ++ |
| C5H9O4N | | | | | | ++ | ++ | | | + |
| C9H1103N | | | | ++ | ++ | ++ | ++ | | | + |
| C9H11O3N | | | | ++ | ++ | ++ | ++ | | | + |
| C9H11O3N | | | | ++ | ++ | ++ | ++ | | | + |
| C4H9O3N | | | | | + | | ++ | | | + |
| C5H10O3N2 | | | | | | ++ | ++ | | | ++ |
| C3H7O2NS | | | | | +++ | +++ | | | | |
| C6H12O4N2S2 | | | | | | ++ | +++ | | | ++ |
| C5H12O2N2 | | | | | | ++ | ++ | | | + |
| C6H14O2N2 | | | | | | ++ | ++ | | | + |
| C6H9O2N3 | | - | | + | ++ | ++ | ++ | + | | ++ |
| C11H12O2N | | | | | | | ++ | | | + |
| C3H8O5NP | + | + | ++ | ++ | ++ | ++ | ++ | ++ | +++ | ++ |
| C4H7O4N | 0 | + | | + | ++ | ++ | ++ | | ++ | ++ |
| C6H9O6N | + | + | | | | | ++ | ++ | | |
| C10H18O7N2 | | | | ++ | ++ | | ++ | | | ++ |
| C10H16O8N2 | + | ++ | ++ | ++ | ++ | ++ | ++ | | +++ | ++ |
| C3H6O3 | | | | | - | - | 0 | | | |
| H3O3B | - | - | | | | | | | ++ | ++ |

Nachfolgend werden exemplarisch die Eigenschaften anorganischer-, Stickstoff- und schwefelfreier organischer Säuren, Zucker, organischer Schwefel-Verbindungen, Aminosäuren, Chelatkomplexbildner und sonstiger Verbindungen als Komplexbildner beschrieben.

Anorganische Komplexbildner:

Das Hydronium-Ion bildet besonders mit den seltenen Erden schwerlösliche Komplexe. Mit allen Nebengruppenelemente der vierten Periode sowie einzelnen Mitgliedern der Bor-Gruppe werden sowohl gutlösliche als auch schwerlösliche Verbindungen gebildet. Als Beispiel kann hier das Kobalt genannt werden.

Folgende Dissoziationsreaktionen kann ungeschütztes Kobalt in Wasser durchführen:

$Co^{2+} + OH^- \leftrightarrow CoOH^+$
pK = 4,3

| | |
|---|---|
| $Co^{2+} + 2\ OH^- \leftrightarrow Co(OH)_2$ | pK = 8,4 |
| $Co^{2+} + 3\ OH^- \leftrightarrow Co(OH)_3^-$ | pK = 9,7 |
| $Co^{2+} + 4\ OH^- \leftrightarrow Co(OH)_4^{2-}$ | pK = 10,2 |
| $2\ Co^{2+} + OH^- \leftrightarrow (Co)_2OH^{3+}$ | pK = 2,7 |
| $4\ Co^{2+} + 4\ OH^- \leftrightarrow (Co)_4(OH)_4$ | pK = 25,6 |
| $Co^{2+} + 2\ OH^- \leftrightarrow Co(OH)_2\ (s) \downarrow$ | pK = 14,9 |

Bei Überschreiten des Löslichkeitsproduktes von $Co(OH)_2$ überwiegt die Fällungsreaktion, da die Aktivität des Feststoffes zu 1 definiert ist und somit nicht mehr von seiner Konzentration abhängt.

$$K = 10^{14,9} = \frac{a(Co(OH)_2)}{a(Co^{2+}) \cdot a(OH^-)^2} = \frac{1}{a(Co^{2+}) \cdot a(OH^-)^2} \quad \Leftrightarrow \quad a(Co^{2+}) \cdot a(OH^-)^2 = 10^{-14,9}$$

Bevor das Löslichkeitsprodukt des Kobalts überschritten wird, reduzieren jedoch die löslichen Kobalthydroxidkomplexe die Konzentration des freien $Co^{2+}$-Ions.

Das Anion der Blausäure ($CN^-$) und dessen Komplexverbindungen, die ebenfalls als Liganden dienen können, bilden zwar sehr stabile Komplexe mit den Nebengruppenelementen der vierten Periode aus, jedoch sind solche Komplexe nicht anaerob abbaubar und deshalb für die Zwecke der Erfindung bei einer anaeroben Fermentation nicht geeignet. Allerdings kann, beispielsweise, die mit der Blausäure engverwandte Form Thiocyanat ($HCS^-$) verwendet werden, da es nicht ganz so stabile Komplexe ausbildet.

Die Sauerstoffverbindungen des Phosphors komplexieren zweiwertige Kationen in hohem Maße. Besonders bevorzugt sind hier Polyphosphate, wie Pyrophosphat und Triphosphat. Pyrophosphat komplexiert sehr stark Magnesium und Mangan, auch in Gegenwart von $Zn^{2+}$, $Fe^{2+}$, $Ni^{2+}$ und $Co^{2+}$, die von den meisten Komplexbildnern bevorzugt gebunden werden.

Borsäure ist dank ihrer Eigenschaft als Lewis-Säure ein sehr guter Komplexbildner für $Fe^{3+}$. Zweiwertige Ionen wie $Ca^{2+}$ und $Mg^{2+}$ werden nur schlecht komplexiert.

Stickstoff- und schwefelfreie organische Säuren:

Die freien flüchtigen Fettsäuren (volatile fatty acids, VFA: Ameisensäure, Essigsäure, Propionsäure, i-, n-Buttersäure, i-, n-Valeriansäure, i-, n-Capronsäure) zeigen nur schwache komplexierende Eigenschaften. $Cu^{2+}$ und $Fe^{3+}$ werden durch VFA komplexiert. $Cu^{2+}$ wird moderat komplexiert; der Komplexierungsgrad der VFA-$Fe^{3+}$-Komplexe sinkt mit steigender Kettenlänge.

Modifizierte kurzkettige Hydroxy- oder Ketofettsäuren zeigen ebenfalls nur leichte Tendenzen zur Bildung von Komplexen. Hydroxyessigsäure (Glycolsäure) 2-Hydroxypropionsäure (Milchsäure), Oxoessigsäure, Oxopropionsäure (Brenztraubensäure, Pyruvat) werden teilweise in erheblichen Mengen in der Zelle gebildet. Komplexe bilden sie in geringen Mengen mit $Cu^{2+}$, und etwas schlechter auch mit $Fe^{2+}$, $Ni^{2+}$ und $Co^{2+}$.

Oxalsäure ist ein moderater Komplexbildner mit $Fe^{2+}$, $Ni^{2+}$, $Co^{2+}$, $Cu^{2+}$ und $Zn^{2+}$ und ein guter Komplexbildner für $Fe^{3+}$, fällt jedoch $Ca^{2+}$ aus der Lösung. Weinsäure, Äpfelsäure und meso-Äpfelsäure zeigen schlechte Komplexierungseigenschaften für zweiwertige Ionen (Ausnahme $Cu^{2+}$), aber gute Komplexierungseigenschaften für dreiwertige Ionen ($Fe^{3+}$, $Al^{3+}$). Citronensäure und in etwas geringerem Maße auch iso-Citronensäure zeigen gute Komplexierungseigenschaften für $Co^{2+}$, $Ni^{2+}$, $Cu^{2+}$ und $Fe^{3+}$. Salizylsäure ist ein guter Komplexbildner für $Zn^{2+}$, ein sehr guter Komplexbildner für $Mn^{2+}$, $Co^{2+}$, $Ni^{2+}$, $Cu^{2+}$ und ein hervorragender Komplexbildner für $Fe^{3+}$. Gluconsäure ist ein moderater Komplexbildner für $Ni^{2+}$-Komplexe.

Zucker:

Von den Zuckern kann die Galakturonsäure, das Monomer der Polygalakturonsäure, einem Grundbaustein des Pektins als nennenswerter Komplexbildner genannt werden. Es ist in der Lage selektiv $Fe^{2+}$ sehr gut zu komplexieren. Andere Hexosen und Pentosen wie z.B. Glucose, Galaktose oder Arabinose zeigen keine großen Neigungen zum Bilden von Komplexen.

Organische Schwefelverbindungen:

Stickstoff- und schwefelfreie organische Säuren - wie oben beschrieben, bei denen ein Sauerstoffatom gegen ein Schwefelatom ausgetauscht wurde, zeigen wesentlich bessere Komplexierungseigenschaften. So sind z.B. Mercaptoessigsäure (Thio-Gycolsäure), Mercaptopropionsäure (Thio-Milchsäure) gute Komplexbildner für $Mn^{2+}$, sehr gute für $Fe^{2+}$, $Co^{2+}$ und hervorragende Komplexbildner für $Fe^{3+}$ und $Zn^{2+}$. Die Mercaptoäpfelsäure unterscheidet sich von der Äpfelsäure in ihrem Komplexierungsspektrum, in dem sie $Ni^{2+}$, $Zn^{2+}$ und in geringerem Maße auch $Co^{2+}$ gut komplexiert. Im Gegensatz zu den bisher genannten organischen Schwefelverbindungen enthält die Thio-diessigsäure keine -SH-Gruppe, sondern eine -S- Ethergruppe. Sie komplexiert $Fe^{2+}$, $Co^{2+}$, $Ni^{2+}$, $Zn^{2+}$ gut, $Cu^{2+}$ und $Al^{3+}$ sehr gut, jedoch kein $Fe^{3+}$.

Aminosäuren:

Aminosäuren sind zum Teil hervorragende Komplexbildner. Sie sind naturgemäß biologisch abbaubar oder können zumindest von der Zelle aufgenommen und verwertet werden. Die Aminosäure Glycin zeigt für $Ca^{2+}$ schlechte und für $Mg^{2+}$ moderate Komplexierungseigenschaften. $Co^{2+}$, $Ni^{2+}$, $Cu^{2+}$ und $Zn^{2+}$ werden sehr gut komplexiert, $Fe^{3+}$ wird hervorragend komplexiert. Alanin und Valin zeigen ähnliche Komplexierungseigenschaften. Sie komplexieren $Ni^{2+}$, $Cu^{2+}$ und $Zn^{2+}$ sehr gut. Leucin komplexiert $Mn^{2+}$ nur moderat, $Cu^{2+}$ und $Zn^{2+}$ jedoch sehr gut. Für Phenylalanin sind sehr gute Komplexierungseigenschaften für $Cu^{2+}$ und $Zn^{2+}$ bekannt. Bei beta-Alanin ist nur eine gute Komplexierungsfähigkeit für $Ni^{2+}$ bekannt. Asparaginsäure komplexiert $Ni^{2+}$, $Cu^{2+}$ und $Zn^{2+}$ sehr gut, $Al^{3+}$ jedoch nur moderat. Glutaminsäure, deren Salz auch als Geschmacksverstärker bekannt ist komplexiert $Ni^{2+}$, $Cu^{2+}$ sehr gut, $Zn^{2+}$ jedoch etwas schlechter. Die ortho-, meta- und para-Isomere des Tyrosins zeigen sehr Ähnliche Eigenschaften hinsichtlich der Komplexbildung. Sie komplexieren $Zn^{2+}$ gut, $Mn^{2+}$, $Ni^{2+}$, $Co^{2+}$ und $Cu^{2+}$ sehr gut. Threonin zeigt gute Komplexierungseigenschaften für $Co^{2+}$ und $Zn^{2+}$, $Cu^{2+}$ wird sehr gut komplexiert. Glutamin zeigt sehr gute Komplexierungseigenschaften für $Ni^{2+}$, $Cu^{2+}$ und $Zn^{2+}$. Cystein zeigt von allen Aminosäuren die besten Komplexierungseigenschaften. Besonders $Co^{2+}$ und $Ni^{2+}$ werden hervorragend von Cystein komplexiert. Auch in seiner oxidierten Form, dem Disulfid Cystin hält es hervorragend $Cu^{2+}$ in Lösung. $Ni^{2+}$ und $Zn^{2+}$ werden immer noch sehr gut komplexiert. Die Aminosäure Omithin, die nicht in Proteinen vorkommt, und Lysin zeigen ähnliche Komplexierungseigenschaften. Sie bilden mit $Ni^{2+}$ und $Cu^{2+}$ sehr gut Komplexe, $Zn^{2+}$ wird gut komplexiert. Histidin zeigt schlechte Komplexierungseigenschaften für $Ca^{2+}$, gute für $Mn^{2+}$ und $Al^{3+}$ und sehr gute für $Co^{2+}$, $Ni^{2+}$, $Cu^{2+}$ und $Zn^{2+}$. Thryphtophan zeigt sehr gute Komplexierungseigenschaften für $Cu^{2+}$ und gute für $Zn^{2+}$. Die Aminosäuren Arginin, Asparagin, Isoleucin, Methionin und Serin sowie die nicht proteinogenen Aminosäuren Homo-Cystein und Homo-Serin können ebenfalls Metalle komplexieren.

Ferner weisen Dipeptide und Tripeptide sehr gute Komplexierungseigenschaften aufweisen (z.B. L-Valyl-L-Valine für $Ni^{2+}$), diese Verbindungen sind jedoch teurer als einfache Aminosäuren.

Chelatkomplexbildner:

Chelatkomplexbildner sind meist tertiäre Amine. Ihre prominentesten Vertreter sind das EDTA (Ethylendiamintetraessigsäure), welches $Mg^{2+}$ gut, $Ca^{2+}$, $Fe^{2+}$, $Mn^{2+}$, $Co^{2+}$, $Ni^{2+}$, $Cu^{2+}$, $Zn^{2+}$ sehr gut und $Fe^{3+}$ hervorragend komplexiert und das NTA (Nitrilotriessigsäure), welches ein ähnliches Komplexierungsspektrum aufweist und identische Prioritäten hat. EDTAist nicht anaerob abbaubar ist und NTA ist krebserregend. Darüber hinaus gibt es aber eine ganze Reihe an weiteren Chelatkomplexbildnern, die diese Nachteile nicht ausweisen. Ethylendiamindibernsteinsäure (EDDS) besitzt Isomere, die biologisch abbaubar sind. Ethylendiiminodiessigsäure (EDDA) komplexiert $Co^{2+}$ und $Zn^{2+}$ sehr gut, $Mn^{2+}$ gut. Ethylenglycolbisaminoethyltetraessigsäure (EGTA) zeigt ein ähnlich gutes Komplexierungsverhalten wie EDTA, weißt jedoch höhere Affinitäten zu $Ca^{2+}$ und $Mg^{2+}$ auf.

Sonstige Verbindungen:

Sonstige Verbindungen, wie die Verbindung Acetylaceton komplexieren durch die di-Keto-Gruppe $Mg^{2+}$, $Mn^{2+}$, $Fe^{2+}$, $Co^{2+}$ moderat, $Ni^{2+}$, $Cu^{2+}$ gut und $Fe^{3+}$ und $Al^{3+}$ hervorragend. Orotsäure, ein mit zwei Stickstoffatomen heterocyclischer Nichtaromat ist ebenfalls in der Lage $Co^{2+}$, $Ni^{2+}$ und $Cu^{2+}$ zu komplexieren. N-Phospomethylglicin ist zwar ein Komplexbildner mit sehr breitem Spektrum, hemmt jedoch die aromatische Aminosäuresynthese und ist als Komplexbildner für die Zugabe in einen Bioreaktor nicht geeignet. Ferner gibt es bekannte Ersatzstoffe wie Zeolithe, die als Molekularsiebe wirken und ebenfalls zur Verbesserung der Bioverfügbarkeit von Spurenelementen eingesetzt werden können.

Es werden erfindungsgemäß Komplexbildner verwendet, die von Mikroorganismen, bevorzugt anaerobe Bakterien, resorbiert werden, wobei (1) die Spurenelemente in komplexierter Form über die Zellmembran transportiert und danach (2) die Spurenelemente in der Zelle freigesetzt werden. Letzteres kann, beispielsweise, durch eine Folgereaktion des Komplexbildners, durch eine Oxidation oder Reduktion des Spurenelements, durch die pH-Verschiebung beim Überqueren der Zellwand oder durch den biologischen Abbau des Komplexbildners erfolgen. Bei einem bakteriellen Verfahren wie dem Biogasverfahren erfolgt der Transport des Spurenelements in korn-plexierter Form über die bakterielle Zellwand und die Zellmembran in das Cytosol der Zelle, wo das Spurenelement freigesetzt wird.

In einer Ausführungsform der erfindungsgemäßen Lösung ist zumindest einer der Komplexbildner biologisch abbaubar; gegebenenfalls sind alle Komplexbildner anaerob abbaubar.

In einer Ausführungsform der Erfindung umfasst die Spurenelementelösung als Komplexbildner zumindest ein tertiäres Amin, beispielsweise EDTA, NTA, EDDS, EDDA; und zumindest einen Komplexbildner ausgewählt aus zumindest einem anorganischen Komplexbildner, zumindest einer Stickstoff-und Schwefelfreien organischen Säure, und Mischungen davon.

Der anorganische Komplexbildner ist bevorzugt eine Sauerstoffverbindung des Phosphors. Die Stickstoff- und Schwefelfreie organische Säure kann, beispielsweise, aus Citronensäure, Iso-Citronensäure, Salizylsäure, Gluconsäure und Mischungen davon ausgewählt werden.

In einer besonders bevorzugten Ausführungsform der Erfindung umfasst die Spurenelementelösung als Komplexbildner EDTA und eine Sauerstoffverbindung des Phosphors, insbesondere, phosphorige Säure oder deren Salze, beispielsweise Polyphosphate wie Pyrophosphat.

In einer anderen bevorzugten Ausführungsform der Erfindung umfasst die Spruenelementelösung als Komplexbildner EDTA und Citronensäure oder ein Salz der Citronensäure.

In einer weiteren Ausführungsform der Erfindung umfasst die Spurenelementelösung als Komplexbildner zumindest eine Sauerstoffverbindung des Phosphors und zumindest einen Komplexbildner gewählt aus tertiären Aminen, Citronensäure, Salze der Citronensäure und Mischungen davon.

Wird zumindest eine Sauerstoffverbindung des Phosphors als Komplexbildner erfinungsgemäß verwendet, kann beispielsweise, phosphorige Säure und Salze davon, insbesondere Polyphosphate wie, beispielsweise Pyrophosphat oder Triphosphat verwendet werden. Vorteilhaft können auch Mischungen verschiedener- Polyphosphate verwendet werden.

Die Verwendung von, Polyphosphaten als Komplexbildner ist vorteilhaft, da damit zugleich auch der Makronährstoff Phosphor als Zuschlagstoff verabreicht wird.

Eine weitere, beispielhafte Kombination der Komplexbildner für die Spurenelementlösung gemäß der Erfindung ist Ethylendiamintetraessigsäure (EDTA), Citronensäure und Catechol. Gegebenenfalls umfasst diese Spurenelementlösung noch weitere Komplexbildner. Gegebenenfalls kann EDTA durch einen anaerob abbaubaren, starken Komplexbildner ausgetauscht werden.

Für bestimmte Verwendungen Ader Erfindung kann es vorteilhaft sein, der Spurenelementlösung weder EDTA, noch NTA oder N-Phosphomethylglicin zuzusetzen, da EDTA nicht anaerob abbaubar, NTA krebserregend und N-Phosphomethylglicin die aromatische Aminosäuresynthese hemmt.

Zu den Spurenelementen, die auch als Spurenmetalle oder Mikronährstoffe bezeichnet werden, zählen Eisen (Fe), Nickel (Ni), Kobalt (Co), Selen (Se), Wolfram (W), Blei (Pb), Kupfer (Cu), Cadmium (Cd), Molybdän (Mo), Wolfram (W), Vanadium (V), Mangan (Mn), und Zink (Zn). Die Spurenelementlösung der Erfindung umfasst zumindest eines dieser Elemente. Die Zusammensetzung der Spurenelementlösung und die Menge des jeweiligen Elements sind abhängig vom verwendeten Substrat und den Mikroorganismen der jeweiligen Fermentation. Für Biogasverfahren umfasst die Spurenelementlösung bevorzugt zumindest Molybdän, Kobalt, Bor und gegebenenfalls Nickel. Letztere Spurenelementlösung ist insbesondere für Maissubstrate vorteilhaft. In Biogasverfahren können Molybdän, Nickel und Kobalt in relativ großen Konzentrationen dem Fermenter zugegeben werden, wodurch die Leistung und der Wirkungsgrad der Fermentation wesentlich verbessert werden. Kobalt, Nickel und Mangan sind nur sehr schwach lösliche Metalle (insbesondere im Vergleich zu Magnesium und Calzium), weshalb die Erhöhung der Bioverfügbarkeit dieser Metalle durch die erfindungsgemäße Spurenelementlösung besonders vorteilhaft ist, da insbesondere Kobalt und Nickel, aber auch Mangan für die Methanoganese essentiell sind.

Zusätzlich zu den Spurenelementen und den Komplexbildnern kann die erfindungsgemäße Lösung ferner weitere Alkali-, Erdalkali- und Schwermetalle; Enzyme, Vitamine, Aminosäuren, Fettsäuren, Kohlenstoff-Quellen, Stickstoffverbindungen und sonstige Nährstoffe, die für den Stoffwechsel der Mikroorganismen im Bioreaktor vorteilhaft sind, umfassen.

Ferner betrifft die Erfindung auch die Verwendung der erfindungsgemäßen Spurenelementelösung für ein Biogasverfahren

Eine Spurenelementlösung, die mindestens zwei oder mehr, der oben beschriebenen Komplexbildner umfasst, ist neben Biogasverfahren auch für andere anaerobe Fermentationen bei neutralen oder schwach saurem pH-Wert verwendbar, bei denen Spurenelemente in Gegenwart von Sulfid-Ionen ausfallen bzw. schwerlösliche Komplexe bilden können.

Als Ausgangssubstrat für Biogasverfahren sind beispielsweise geeignet: Fermentierbare Reststoffe wie Klärschlamm, Bioabfall oder Speisereste; Dünger wie Gülle oder Mist; sowie nachwachsende Energiepflanzen wie Mais, Getreide oder Gras.

Die erfindungsgemäße Spurenelementlösung wird bei Biogasverfahren mit Monosubstraten aus pflanzliche Rohstoffen verwendet.

Gegebenenfalls kann auch zumindest ein Komplexbildner oder eine erfindungsgemäße Mischung der Komplexbildner zusätzlich zur erfindungsgemäßen Spurenelementelösung dem Biogasverfahren zugefügt werden. Bei Biogasverfahren, die Eisen-Magnesium- oder Calzium-reiche Substrate umsetzen, ist es vorteilhaft die erfindungsgemäßen Komplexbildner im Überschuss zuzugeben, um das in Verbindung mit der Tabelle 2 beschriebene Phänomen der Verdrängung von Kobalt, Nickel und Zink durch Magnesium und/oder Kalzium zu verhindern. Bevorzugt wird hierfür zusätzlich zur erfindungsgemäßen Spurenelementelösung eine erfindungsgemäße Mischung der Komplexbildner der Biogasfermentation zugeschlagen.

So kann die Spurenelementlösung für Biogasverfahren verwendet werden, die mit Monosubstraten ausschließlich auf Basis von pflanzlicher Biomasse, aus der landwirtschaftlichen Produktion betrieben werden. Ein derartiges Verfahren benötigt keine Co-Substrate in der Form tierischer Exkremente, beispielsweise Gülle, Stallmist oder Trockenkot. Das Monosubstrat für die Vergärung kann auch eine Mischung verschiedener Arten von Aufbereitungen des gleichen Substrats sein, z.B. eine Mischung aus Maissilage, Maiskörnern und Frischmais. Alternativ dazu können natürlich auch Mischungen aus verschiedenen pflanzlichen Substraten, z.B. aus Mais und Gras, fermentiert werden.

Als Monosubstrate sind pflanzliche Produkte und/oder Abfälle geeignet. Dazu zählen Grasschnitt, Silage, Energiepflanzen, als "nachwachsende Rohstoffe" (NAWRO) bezeichnete Pflanzen, Lagerungsreste, Erntrückstände oder pflanzliche Abfälle. Beispiele für als Substrate geeignete Pflanzen sind: Mais, Roggen, Gras, Rübe, Sonnenblume und Raps.

Bei Versuchen mit Maissilage wurde überraschend gefunden, dass durch Zusatz einer Spurenelementlösung gemäß der Erfindung die Fermentierbarkeit des Substrats verbessert wurde. Zudem konnte durch die zusätzliche Zugabe von Phosphat zu dem Substrat aus Maissilage eine deutliche Steigerung der Gasproduktion erreicht werden, wobei die hydraulische Verweilzeit des Substrats gesenkt wurde. Dadurch konnte die Raumbelastung des Fermenters um etwa das zehnfache von etwa 1,5 kg auf etwa 10 $kg_{oTM}/(m^3\ d)$ gesteigert werden. Im Pflanzenmaterial stehen organisch gebundener Phosphor sowie die Spurenelemente nur limitiert für die Methanvergärung zur Verfügung. Deshalb können der Umsatz der an der Vergärung beteiligten Bakterien durch Zusatz der Spurenelementlösung maßgeblich erhöht und dabei die Verwertung des pflanzlichen Substrats verbessert und dadurch wiederum die Gärrückstände im Bioreaktor verringert werden.

Besonders vorteilhaft ist die erfindungsgemäße Spurenelementelösung für $Mg^{2+}$-und/oder $Ca^{2+}$-reiche Fermentationssubstrate, da durch die zumindest zwei verschieden starken Komplexbildner eine ausreichende Löslichkeit bzw. Bioverfügbarkeit der gering löslichen Mikronährstoffe wie Kobalt, Nickel und Mangan bereitgestellt wird, trotz der erhöhten Löslichkeit von Magnesium und, gegebenenfalls Calzium, unter den Bedingungen der Biogasfermentation.

Zur Erfindung gehört auch ein Verfahren zur Gewinnung von Biogas in einer Biogas-anlage, wobei während der Fermentation die Spurenelementlösung in den Fermenter zur Biogasgewinnung eingeleitet wird

Gegebenenfalls können die Spurenelemente und die Komplexbildner auch in trockener, z.B. lyophilisierter oder pulvriger, Form bereitgestellt werden, um erst unmittelbar vor der Zuleitung in den Fermenter in Lösung gebracht zu werden. Die Zudosierung der Spurenelementlösung in den Fermenter kann batchweise, diskontinuierlich oder kontinuierlich erfolgen.

Nachfolgend wird die Erfindung mit die die Erfindung nicht einschränkenden Figuren und Beispielen illustriert:

Es zeigen

Figur 1    Zugabe einer komplexierten Spurenelementlösung in einen 500 $m^3$ Biogasreaktor mit Maissilage nach Beispiel 3. Die Zugabe startet bei beginnender Versäuerung des Reaktors bei einer Raumbelastung von 3 $kg_{oTM}/(m^3\ d)$. Durch die Zugabe von bioverfügbaren Spurenelementen kann die Raumbeladung auf 10 $kg_{oTM}(m^3\ d)$ gesteigert werden, ohne dass flüchtige Fettsäuren im Reaktor akkumulieren,

Figur 2    Daten der Tabelle 2:

(a) Konzentration der Spurenelemente in Gärrest komplexiert mit 50% NTA und einer Spurenelementzusammensetzung gemäß Tabelle 5,
(b) Konzentration der Spurenelemente in Gärrest komplexiert mit 50% NTA und hundertfache Spurenelement-Einsatzmenge der Tabelle 5.
(c) Konzentration der Spurenelemente in Gärrest komplexiert mit 50% NTA und einfache Menge Fe aus Tabelle 5 und hundertfache Menge der anderen Spurenelemente aus Tabelle 5.

Figur 3    Daten der Tabelle 3:

(a) Konzentration der Spurenelemente in Gärrest komplexiert mit 50%NTA nach Zugabe von Störstoffen (500%Fe(III)),
(b) Konzentration der Spurenelemente in Gärrest komplexiert gemäß der Erfindung (100% EDTA, 1000% Phosphorsäurenmischung) nach Zugabe von Störstoffen (500% Fe(III)

**Beispiel 1:** Komplexierung der Spurenelementlösung des DSMZ-Mediums 141

Die Zusammensetzung der Spurenelementelösung ist in Tabelle 5 angegeben. Bemerkenswert ist auch hier, dass laut Literaturangaben die Konzentration der mit Sulfid fällbaren Ionen deutlich höher ist, als die Konzentration des Komplexbildners NTA. Beim Einsatz dieser Spurenelementlösung bildet sich auch erwartungsgemäß ein feiner Niederschlag aus, sobald ein Reduktionsmittel auf Schwefelbasis ($Na_2S$; $Na_2S_2O_3$) hinzugegeben wird. Dies kann durch eine entsprechende Zugabe an Komplexbildnern z.B. 15 mmol/L Pyrophosphat, 0,2 mmol/L Galakturonsäure, 0,4 mmol/L Cystein, 0,05 mmol/L Acetylaceton und 0,3 mmol/L Leucin unterbunden werden.
Tabelle 5: Zusammensetzung der Spurenelementelösung des Mediums 141 der DSMZ für ein methanogenes Archaeon

Tabelle 5

|  | m [g/L] | C [mmol/L] |
|---|---|---|
| NTA | 1,5000 | 7,853 |
| $MgSO_4$ x 7 $H_2O$ | 3,0000 | 13,717 |
| $MnSO_4$ x 2 $H_2O$ | 0,5000 | 2,277 |
| NaCl | 1,0000 | 21,739 |
| $FeSO_4$ x 7 $H_2O$ | 0,1000 | 0,199 |
| $CoSO_4$ x 7 $H_2O$ | 0,1800 | 0,339 |
| $CaCl_2$ x 2 $H_2O$ | 0,1000 | 0,500 |
| $ZnSO_4$ x 7 $H_2O$ | 0,1800 | 0,306 |
| $CuSO_4$ x 5 $H_2O$ | 0,0100 | 0,022 |
| $KAl(SO_4)_2$ x 12 $H_2O$ | 0,0200 | 0,032 |
| $H_3BO_3$ | 0,0100 | 1,429 |

(fortgesetzt)

| | m [g/L] | C [mmol/L] |
|---|---|---|
| Na$_2$MoO$_4$ x 2 H$_2$O | 0,0100 | 0,087 |
| NiCl$_2$ x 6 H$_2$O | 0,0250 | 0,047 |
| Na$_2$SeO$_3$ x 5 H$_2$O | 0,0003 | 0,002 |

**Beispiel 2:**

In Tabelle 6 ist eine beispielhafte Zusammensetzung einer Spurenelementelösung gemäß der Erfindung dargestellt. Als erster starker Komplexbildner wird EDTA und als zweiter Komplexbildner eine Mischung von phosphorigen Säuren eingesetzt. Falls das Substrat ein Abfall oder pflanzlicher Rohstoff ist, kann die Lösung in einem Verhältnis von, beispielsweise, 1:100 dem Substrat zudosiert werden.

Tabelle 6

| Element | mmol/L | |
|---|---|---|
| Mo | 0,42 | |
| Ni | 1,12 | |
| Se | 0,08 | |
| W | 0,90 | |
| Mn | 0,80 | |
| Co | 1,00 | |
| Zn | 0,74 | |
| Cu | 0,59 | |
| B | 1,64 | |
| Fe | 4,60 | |
| | | |
| Komplexbildner | mmol/L | mg/L |
| EDTA | 7,2 | 2102 |
| H4P2O7 | 7,2 | 1282 |
| H6P4O13 | 7,2 | 2434 |
| H4P4O12 | 7,2 | 2304 |
| H3PO2 | 7,2 | 475 |
| H3PO3 | 7,2 | 590 |

**Beispiel 3:** Trockenfermentation von Maissilage in einer 500 kW Anlage

In einer Anlage, die nach DE102005041798 ausgeführt ist, wird Maissilage vergoren und zu Biogas umgewandelt. Zu Beginn der Fütterung wird eine volumenspezifische Beladungsrate von 0,75 kg$_{oTM}$/(m$^3$ d) eingestellt und die Fütterung jede Woche um 0,5 kg$_{oTM}$/(m$^3$ d) erhöht. Bei Erreichen einer volumenspezifischen Beladungsrate von 3 kg$_{oTM}$/(m$^3$ d) beginnen die Säuren im Reaktor zu steigen - Ein Zeichen für die Überlastung der anaeroben Biomasse im Reaktor. Die Erhöhung der Fütterung wird zunächst ausgesetzt, der Anstieg der Säuren schreitet jedoch weiter voran. Dem Reaktor wird nun eine handelsübliche Spurenelementlösung zugegeben, die nach der in der Erfindung beschriebenen Methode mit zwei verschieden starken Komplexbildnem komplexiert ist. Die Säuren sinken daraufhin binnen 10 Tagen wieder ab und die Fütterung wird fortgesetzt. Knapp 90 Tage nach dem Beginn der kontinuierlichen Spurenelementzugabe steigen die Säuren erneut an. Die volumenspezifische Beladungsrate liegt mittlerweile bei 7 kg$_{oTM}$/(m$^3$ d). Die

Fütterungrate wird daraufhin für eine Woche halbiert und die zehnfache Tagesdosis An Spurenelementen hinzugegeben. Die Fütterung wird nach einer Woche wieder auf den alten Wert eingestellt und weiter gesteigert. Der Reaktor erreicht seinen Auslegungswert bei 10 $kg_{oTM}/(m^3\ d)$. Die Säurekonzentration liegt mit 1000 mg/L unter der Obergrenze für den Technologiebonus nach dem EEG von 2000 mg/L. Erst die Zugabe der komplexierten Spurenelementlösung ermöglicht die Erhöhung der volumenspezifischen Beladungsrate von 5 (Stand der Technik) auf 10 $kg_{oTM}/(m^3\ d)$. Die Trockenfermation wurde nach einem bekannten Verfahren durchgeführt (Ergebnisse des Biogas-Messprogramms, 2005, Fachagentur für Nachwachsende Rohstoffe, Kapitel 7.3) dokumentiert.

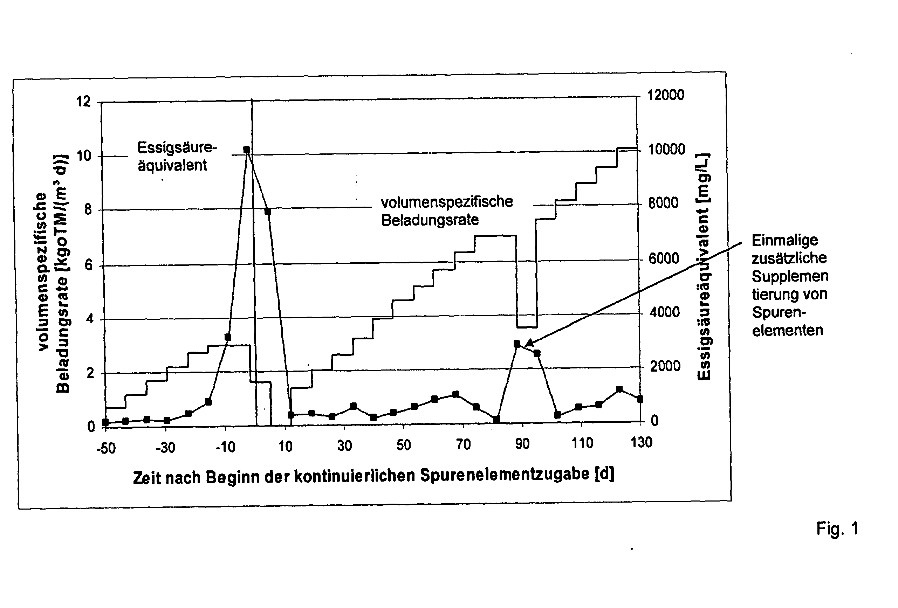

Die Zugabe der erfindungsgemäß komplexierten Spurenelementlösung in einen 800 $m^3$ Biogasreaktor mit Maissilage ist in Figur 1 dargestellt. Die Zugabe startet bei beginnender Versäuerung des Reaktors bei einer Raumbelastung von 3 $kg_{oTM}/(m^3\ d)$. Durch die Zugabe von bioverfügbaren Spurenelementen kann die Raumbeladung auf 10 $kg_{oTM}/(m^3\ d)$ gesteigert werden, ohne dass flüchtige Fettsäuren im Reaktor akkumulieren.

Der Fermentationsrückstand war als Düngemittel geeignet (As<01 mg/kg TM, Pb 2,42 mg/kg TM, Ca 0,28 mg/kg TM, Cr 8,96 mg/kg TM, Ni 6,05 mg/kg TM, Hg 0,08 mg/kg TM, TI <0,2 mg/kg TM, Se<0,5 mg/kg TM, Cu 3 mg/kg FM, Zn 10 mg/kg FM, B 0,8 mg/kg FM, Co 0,072 mg/kg FM; TM = Trockenmasse, FM = Frischmasse).

## Patentansprüche

1.  Verwendung einer Spurenelementlösung zum Supplementieren eines Monosubstrats aus pflanzlicher Biomasse aus der landwirtschaftlichen Produktion oder Mischungen aus verschiedenen pflanzlichen Substraten mit Spurenelementen für eine anaerobe Biogasfermentation, wobei

    (a) die Spurenelementlösung zumindest ein Spurenelement und zumindest zwei Komplexbildner mit verschiedenen Affinitäten für Metallionen umfasst,
    (b) das zumindest eine Spurenelement ein Spurenmetall ausgewählt aus der Gruppe bestehend aus Fe, Ni, Co, Se, W, Pb, Cu, Cd, Mo, V, Mn und Zn ist;
    (c) zumindest ein stärkeren Komplexbildner ein tertiäres Amin ist;
    (d) zumindest ein schwächerer Komplexbildner ausgewählt ist aus der Gruppe bestehend aus einer unorganischen Sauerstoffverbindung des Phosphors, Citronensäure, Iso-Citronensäure, Salizylsäure, Gluconsäure und Mischungen davon, wobei die Sauerstoffverbindung des Phosphors phosphorige Säure, Salze der phosphorigen Säure oder Polyphosphate sind;
    (e) die Komplexbildner in zumindest äquimolarer Menge zu den Spurenelementen vorliegen, und
    (f) der schwächere Komplexbildner in einer vielfachen molaren Menge des stärkeren Komplexbildners vorliegt.

2.  Verwendung nach Anspruch 1, wobei mehr als zwei verschiedene Komplexbildner verwendet werden.

3.  Verwendung nach einem der Ansprüche 1-2, wobei das tertiäre Amin Ethylendiamintetraessigsäure (EDTA) Diethylentriaminpentaessigsäure (DTPA), Hydroxyethylendiamintriessigsäure (HEDTA), Ethylendiamindibemsteinsäure (EDDS), Ethylendiiminodiessigsäure (EDDA) oder Nitrilotriessigsäure (NTA) ist.

4.  Verwendung nach einem der Ansprüche 1-2, wobei als Komplexbildner Ethylendiamintetraessigsäure (EDTA) und Citronensäure oder ein Salz der Citronensäure umfasst sind.

5.  Verwendung nach einem der Ansprüche 1-2, wobei als Komplexbildner Ethylendiamintetraessigsäure (EDTA) und zumindest eine phosphorige Säure oder deren Salze umfasst sind.

6.  Verwendung nach einem der Ansprüche 1-5, wobei das pflanzliche Substrat Grasschnitt, Silage, Energiepflanzen, als "nachwachsende Rohstoffe" (NAWARO) bezeichnete Pflanzen, Lagerungsreste, Ernterückstände oder pflanzliche Abfälle ist.

7.  Verwendung nach einem der Ansprüche 1-6, wobei die Spurenelementlösung zumindest die Spurenelemente Molybdän, Kobalt und Bor enthält.

8.  Verwendung nach eine der Ansprüche 1-6, wobei die Spurenelementlösung zumindest die Spurenelemente Kobalt, Nickel und Mangan umfasst.

9.  Verwendung nach einem der Ansprüche 1-8, wobei der Biogasfermentation eine größere Menge an Komplexbildnern zugefügt wird, als zur vollständigen Komplexierung der Spurenelemente in der Spurenelementlösung notwendig ist.

10. Verfahren zur Gewinnung von Biogas in einer Biogasanlage, wobei der Biogasanlage eine Spurenelementlösung wie in einem der Ansprüche 1-5 definiert, während der Fermentation zugegeben wird.

**Claims**

1. Use of a trace element solution to supplement a monosubstrate of vegetable biomass from agricultural production or blends of different vegetable substrates with trace elements for an anaerobic biogas fermentation, wherein

   (a) the trace element solution includes at least one trace element and at least two complexing agents with different affinities for metal ions
   (b) the trace element or elements is or are a trace metal chosen from the group comprising Fe, Ni, Co, Se, W, Pb, Cu, Cd, Mo, V, Mn and Zn
   (c) at least one stronger complexing agent is a tertiary amine
   (d) at least one weaker complexing agent is chosen from the group comprising an inorganic oxygen compound of phosphorus, citric acid, isocitric acid, salicylic acid, gluconic acid and mixtures thereof, wherein the oxygen compound of phosphorus is phosphoric acid, salts of phosphoric acid or polyphosphate
   (e) the complexing agents are present in amounts which are at least equimolar to the trace elements, and
   (f) the weaker complexing agent is present in a molar amount which is many times that of the stronger complexing agent.

2. Use according to claim 1, wherein more than two different complexing agents are used.

3. Use according to any of claims 1 or 2, wherein the tertiary amine is ethylenediamine tetra acetic acid (EDTA), diethylenetriamine pentaacetic acid (DTPA), hydroxyethylene diamine triacetic acid (HEDTA), ethylenediamine disuccinic acid (EDDS), ethylenediimino diacetic acid (EDDA) or nitrilotriacetic acid (NTA).

4. Use according to any of claims 1 or 2, wherein ethylenediamine tetra acetic acid (EDTA) and citric acid or a salt of citric acid are included as complexing agents.

5. Use according to any of claims 1 or 2, wherein ethylenediamine tetra acetic acid (EDTA) and at least one phosphoric acid or its salts are included as complexing agents.

6. Use according to any of claims 1 to 5, wherein the vegetable substrate is cut grass, silage, energy crop plants, plants described as "renewable raw materials" (NAWRO), storage residues, crop residues or vegetable waste.

7. Use according to any of claims 1 to 6, wherein the trace element solution contains at least the trace elements molybdenum, cobalt and boron.

8. Use according to any of claims 1 to 6, wherein the trace element solution contains at least the trace elements cobalt, nickel and manganese.

9. Use according to any of claims 1 to 8, wherein a larger quantity of complexing agents is added to the biogas fermentation than is necessary for complete complexing of the trace elements in the trace element solution.

10. Process for obtaining biogas in a biogas plant, wherein a trace element solution as defined in claims 1 - 5 is fed into the biogas plant during fermentation.

**Revendications**

1. Utilisation d'une solution d'oligo-éléments pour compléter un monosubstrat de biomasse végétale provenant d'une production agricole ou de mélanges provenant de divers substrats végétaux avec des oligo-éléments pour une fermentation de biogaz anaérobie, dans laquelle

   (a) la solution d'oligo-éléments comprend au moins un oligo-élément et au moins deux formateurs de complexes avec différentes affinités pour des ions métalliques,
   (b) ledit au moins un oligo-élément est un métal choisi dans le groupe constitué de Fe, Ni, Co, Se, W, Pb, Cu, Cd, Mo, V, Mn et Zn ;

(c) au moins un formateur de complexe plus fort est une amine tertiaire ;

(d) au moins un formateur de complexe plus faible est choisi parmi le groupe constitué d'un composé oxygéné anorganique de phosphore, acide citrique, acide isocitrique, acide salicylique, acide gluconique et leurs mélanges, dans lequel le composé oxygéné de phosphore comprend des acides phosphoriques, des sels d'acides phosphoriques ou des polyphosphates ;

(e) les formateurs de complexes se présentent dans une quantité au moins équimolaire par rapport aux oligo-éléments, et

(f) le formateur de complexe plus faible se présente sous une quantité multi-molaire du formateur de complexe plus fort.

2. Utilisation selon la revendication 1, dans lequel on utilise plus de deux formateurs de complexes différents.

3. Utilisation selon l'une des revendications 1 ou 2, dans laquelle l'amine tertiaire est de l'acide éthylène diamine tétraacétique (EDTA), de l'acide diéthylène triamine pentaacétique (DTPA), de l'acide hydroxy éthylène diamine triacétique (HEDTA), de l'acide éthylène diamine disuccinique (EDDS), de l'acide éthylène diimine diacétique (EDDA) ou de l'acide nitrile triacétique (NTA).

4. Utilisation selon l'une des revendications 1 ou 2, dans lequel on inclut à titre de formateur de complexe de l'acide éthylène diamine tétraacétique (EDTA) et de l'acide citrique ou un sel d'acide citrique.

5. Utilisation selon l'une des revendications 1 ou 2, dans lequel on inclut à titre de formateur de complexe de l'acide éthylène diamine tétraacétique (EDTA) et au moins un acide phosphorique ou des sels de celui-ci.

6. Utilisation selon l'une des revendications 1 à 5, dans lequel le substrat végétal est constitué d'herbe coupée, d'ensilage, de plantes énergétiques, de plantes dénommées "matières premières renouvelables", de restes de stockage, de restes de récolte ou de déchets végétaux.

7. Utilisation selon l'une des revendications 1 à 6, dans laquelle la solution d'oligo-éléments contient au moins les oligo-éléments de molybdène, de cobalt et de bore.

8. Utilisation selon l'une des revendications 1 à 6, dans laquelle la solution d'oligo-éléments contient au moins les oligo-éléments de cobalt, de nickel et de manganèse.

9. Utilisation selon l'une des revendications 1 à 8, dans laquelle on ajoute à la fermentation de biogaz une quantité plus importante de formateurs de complexes que celle qui serait nécessaire pour la formation complète de complexes d'oligo-éléments dans la solution d'oligo-éléments.

10. Procédé pour l'obtention de biogaz dans une installation de biogaz, dans lequel on ajoute dans l'installation de biogaz une solution d'oligo-éléments telle que définie dans l'une des revendications 1 à 5 pendant la fermentation.

Fig. 1

Fig. 2a

Fig. 2b

Fig. 2c

Fig. 3a

Fig. 3b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10300082 A1
- DE 10139829 A1
- DE 10226795 A1
- EP 0328560 B1
- AT 413209 B
- US 5342524 A

**In der Beschreibung aufgeführte Nicht-Patentliteratur**

- **GONZALEZ-GIL G. et al.** *Biotechnology and Bioengineering,* 2003, vol. 82 (2), 134-142
- **HU et al.** *Biochemical Engineering,* 2007, vol. 38 (1), 98-104
- **CALLANDER IJ et al.** *Biotechnology and Bioengineering,* 1983, vol. 25 (8), 1959-1972
- **RINTALA J. et al.** *Water Science and Technology,* 1991, vol. 24 (3-4), 149-160